# EUROPEAN PATENT APPLICATION

(11) **EP 4 388 870 A1**
(43) Date of publication of application: **26.06.2024**
(21) Application number: 22216276.0
(22) Date of filing: 23.12.2022
(51) Int. Cl.: A01K 67/033, A23K 10/20

(54) **SYSTEM AND METHOD FOR PRODUCING AN ANIMAL FEED COMPOSITION COMPRISING WORMS**

(71) Applicant: Corbiota GmbH, 40215 Düsseldorf (DE)
(72) Inventor: Rohde, Julia Katrin, 68169 Mannheim (DE); Kühn, Thorsten, 68169 Mannheim (DE); Boche, Claus-Andreas, 40212 Düsseldorf (DE); Alzer, Markus, 40212 Düsseldorf (DE)
(74) Representative: Eisenführ Speiser

(57) **Abstract**

The invention relates to a method for producing a feed composition, comprising, essentially consisting of or consisting of one or more worm(s), the method comprising the steps:
i) providing one or more worm(s) and/or providing one or more cocoon(s) of one or more worm(s),
ii) placing the worm(s) and/or cocoon(s) provided in step i) on or within a substrate,
iii) raising the provided worm(s) and/or worm(s) hatched from the cocoon(s),
wherein raising includes providing worm feed to the one or more worm(s),
iv) automatically determining at least one average property of worms present in the substrate,
v) automatically adjusting at least one climate parameter to control an ambient climate of the substrate in dependence on the determined average property of worms in the substrate, and
vi) collecting the, one, two, three or more or all worm(s) to obtain the feed composition.

## Description

### FIELD OF THE INVENTION

The present invention relates to a method for producing a feed composition comprising one or more worm(s), to a system for producing a feed composition, to a computer program for controlling a temperature and/or humidity and/or pH value of a substrate suitable for raising worm(s) and/or worm(s) hatched from cocoon(s), and to a non-transitory computer readable data medium storing such a computer program.

### BACKGROUND OF THE INVENTION

Complete protein is a food source of protein containing all of the essential amino acids in adequate proportion for the human diet. Among the complete proteins, meat, eggs and milk are considered to belong to the highest-quality protein sources. Meat, especially from the cattle and poultry industry, is considered as one of the most important sources of complete protein. For example, in poultry industry, broiler chickens are the animals most relied on as source of meat and therefore as complete proteins.

The consumption of meat worldwide, especially mast poultry, is dramatically increasing, and the amount needed to feed the billions of people worldwide in the future is constantly rising. Furthermore, poultry is the only meat accepted for food by every culture and religion on our planet.

Typically, during the breeding and raising of animals for slaughter, the animals are reared with particularly high hygienic standards. In some cases, as for example in modern broiler production, chickens hatch in artificial incubators.

Consequently, animals raised under such conditions have a reduced count of microorganisms, particularly those microorganisms with beneficial effects, when raised under such conditions. Such microorganisms with beneficial effects typically include *Akkermansia muciniphila, Lactobacillaceae* such as *Lactobacillus coleohominis* or *Lactobacillus reuteri, Faecalibacterium prausnizii, Prevotella* and/or *Bifidobacteriaceae* such as *Bifidobacter pseudolongum.*

The reduced count of these microorganisms negatively affects the processing and uptake of nutrients provided by the animal feed in the animals' intestines. Thus, from the same amount of animal feed, these animals receive less nutrients than animals raised under natural conditions. Therefore, either the amount of feed would need to be increased, which typically provides economic and ecological disadvantages, or the growth rate of these animals will be reduced, compared to animals raised under natural conditions.

This problem has been addressed by adding earthworms to animal feed compositions. Earthworms belong to natural feed sources, for example for avian species, including chickens. Nevertheless, earthworms can be added to animal feed for a variety of animals.

Earthworms consume plant material, which comprises cells with a cell wall. For the digestion of constituents of the plant cell wall, earthworms are dependent on microorganisms and have a relatively high microbial activity in their gut. Thus, earthworms may be considered as 'natural' microbiota-sources for animal feed.

Consequently, feeding earthworms to animals raised for slaughter or adding earthworms to their feed provides several advantages. For raising animals on industrial scale, large amounts of worms, preferably earthworms, are required. However, it was found that worm breeding is seasonal and thus depends on factors such as temperature and humidity.

For a worm production on industrial scale, it should thus be possible to produce worms throughout the whole year, without an influence of the current season. Further, it should be possible to provide predictable output quantities, i.e. amounts of produced worms. Importantly, the amount of produced worms needs to be adapted to the respective needs. On one side, a sufficient quantity needs to be produced, whereas at the same time, an excess production needs to be avoided for not wasting energy and other resources. Thus, it is required to provide a sustainable approach with which the rates of production can be increased and decreased, depending on the particular needs.

Furthermore, worms typically harbour a broad variety of microorganisms, including microorganisms, which are harmful for the animals for slaughter, as well. These undesired microorganisms may include, for example, Enterotoxigenic Escherichia coli (ETEC), preferably Enterotoxigenic Escherichia coli (ETEC): F4 (K 88); Clostridium spp., preferably Clostridium perfringens, Clostridium difficile; Brachyspira hyodysenteriae; Lawsonia intracellularis; Campylobacter spp.; Salmonella spp., preferably S. typhimurium, S. coleraesuis; Brucella suis; Histomonas meleagridis; Streptococcus spp., Coliforms, Bacterioides, Eimeria and/or Avian Influenza-A virus.

If worms, which are currently infected by one or more of these pathogens, are added to a feed composition, there is the risk that these pathogens are transferred to the animals fed with the feed composition.

Indeed, during the breeding and raising of animals for slaughter, high rates of microbial infection can be observed, which can provide high rates of death especially among young animals. One example of microbial infection is pododermatitis, which occurs after an initial minor injury, and may progress to a severe infection destroying skin, tendon or bone. Pododermatitis is frequently observed in chickens, ducks or geese, or, respectively, chicklets, ducklings or goslings. Moreover, conditions such as enteric colibacillosis, necrotic enteritis, swine dysentery, Porcine Intestinal Adenomatosis (PIA), diarrhea are frequently observed in animals raised for slaughter and should be avoided. Typically, these conditions are caused by microorganisms such as *Enterotoxigenic Escherichia coli (ETEC),* preferably *Enterotoxigenic Escherichia coli (ETEC): F4 (K 88); Clostridium spp.,* preferably *Clostridium perfringens, Clostridium difficile; Brachyspira hyodysenteriae; Lawsonia intracellularis; Campylobacter spp.; Salmonella spp.,* preferably *S. typhimurium, S. coleraesuis; Brucella suis; Histomonas meleagridis; Streptococcus spp., Coliforms, Bacterioides, Eimeria* and *Avian Influenza-A virus.*

Of course, microbial infections and a high death rate shall be avoided. Currently, the industry tries to counteract the high death rate by providing high amount of medication. Furthermore, the animal food is supplemented with certain microorganisms, which shall be provided to the fed animals. However, it is known that the massive provision of medication, particularly antibiotics, to animals for slaughter bears the risk that the medication is still present in, e.g., meat and meat products obtained from the animals, which in turn leads to negative consequences for the consumers of the meat and meat products. As an example, antibiotics in meat and meat products obtained from animals increase the risk of antibiotic resistance in humans as well as the risk of obtaining antibiotic-resistant pathogens such as MRSA (Methicillin-resistant Staphylococcus aureus) or bacteria producing ESBL (Extended-Spectrum-Betalaktamases, which are enzymes that may cleave and thus destruct antibiotics).

Thus, it would be advantageous to provide an approach, with which it is possible to reduce the amounts or avoid the presence of the above or some of the above microorganisms in the produced worms.

Currently, worms are produced for fishing and compost purposes only. Therefore, the present production processes do not need to fulfil the high standards as described above. Likewise, a lower level of output management is required. Thus, the current worm production processes are predominantly done manually and are very labour intense. In addition, such processes do not fulfil standards, which are required for producing worms free of specific pathogens.

In such methods, adult worms are typically kept in boxes with substrate and fed on a regular basis. Once there is a sufficient amount of cocoons in the box, which is evaluated manually by a worker, the adult worms get separated from the cocoons. Then, the cocoons are put in boxes with substrate and the breeding starts. When a worker evaluates that enough worms have hatched, the hatched worms get separated and are put in new boxes with substrate in which they are fed on a regular basis. All separation steps are usually performed with a drum screen. A worker regularly checks the size of the growing worms. From time to time, the boxes get sieved to separate worms that are ready for sale from worms that still need to grow. All worms that are ready for sale get hand-picked and are then shipped.

In such a process, the output quantity and the timing are left to chance. Decisions are purely based on human experience and judgement. Therefore, such a worm production process is not feasible for producing worms, particularly worms free of specific pathogens, on an industrial scale. In addition, there is no possibility of a full year process for breeding and growing.

### SUMMARY OF THE INVENTION

It is thus a primary object of the present invention to provide a method for producing a feed composition comprising one or more worm(s), which fulfils the above requirements without the (or some of the) above disadvantages.

The primary object of the present invention is solved by a method for producing a feed composition, comprising, essentially consisting of or consisting of one or more worm(s), the method comprising the steps:
i) providing one or more worm(s) and/or providing one or more cocoon(s) of one or more worm(s),
ii) placing the worm(s) and/or cocoon(s) provided in step i) on or within a substrate,
iii) raising the provided worm(s) and/or worm(s) hatched from the cocoon(s),
   wherein raising includes providing worm feed to the one or more worm(s),
iv) automatically determining at least one average property of worms present in the substrate,
v) automatically adjusting at least one climate parameter to control an ambient climate of the substrate in dependence on the determined average property of worms in the substrate, and
vi) collecting, preferably, automatically collecting, the, one, two, three or more or all worm(s) to obtain the feed composition.

It was found that with the method according to the invention, worms can be produced throughout the year. Further, it was found that with the method according to the invention, a predictable and/or adjustable quantity of worms can be produced with high efficiency. In this regard, it is possible to provide a standardized approach for the industrial production of worms.

Preferably, the term "worm", as used herein, refers to earthworms. Particularly preferably, the term "worm", as used herein refers to earthworms selected from the group consisting of *dendrobena veneta, lumbricus terrestris, eisenia fetida,* eudrilus eugeniae, *perionyx excavatus* and *eisenia andreii.* Further preferably, the term "worm", as used herein refers to earthworms selected from the group consisting of *dendrobena veneta, eisenia andrei* and *eisenia fetida.* Even further preferably, the term "worm", as used herein refers to earthworms of the species *dendrobena veneta.*

Preferably, the term "worm", as used herein, refers to a living worm.

Preferably, the term "size of a worm", as used herein, refers to the length of a worm.

Preferably, the, one, two, three or more or all of the one or more worm(s) obtained by a method according to the invention, preferably collected in step vi) of the method according to the invention, has/have an average length in the range of 3.5 cm to 8 cm, and a minimum length of 2 cm and a maximum length of 12 cm. More preferably, the, one, two, three or more or all of the one or more worm(s) has/have an average length in the range of 4 cm to 7 cm, and a minimum length of 3 cm and a maximum length of 11 cm. Even more preferably, the, one, two, three or more or all of the one or more worm(s) has/have an average length in the range of 4 cm to 7 cm, and a minimum length of 3 cm and a maximum length of 10 cm. Further preferably, the, one, two, three or more or all of the one or more worm(s) has/have an average length in the range of 5 cm to 7 cm, and a minimum length of 4 cm and a maximum length of 10 cm. Particularly preferably, the, one, two, three or more or all of the one or more worm(s) has/have an average length in the range of 5 cm to 7 cm, and a minimum length of 4 cm and a maximum length of 9 cm.

Preferably, the, one, two, three or more or all of the one or more worm(s) obtained by a method according to the invention, preferably collected in step vi) of the method according to the invention, has/have an average weight in the range of from 0.25 to 0.75 g, preferably in the range of from 0.3 to 0.7 g, preferably in the range of from 0.4 to 0.6 g. A target average weight may be approximately 0.5 g.

Preferably, the term "average length" and/or "average weight" refers to the average over a plurality of worms.

Preferably, automatically determining an average property of worms present in the substrate in step iv) comprises determining an average amount of the worms, as described herein, in particular determining a number of worms in the substrate and/or an average size of the, one, two, three or more or all worm(s) in the substrate and/or an average weight of the, one, two, three or more or all worm(s) in the substrate.

Preferably, a climate parameter that is adjusted in step v) is a temperature of the substrate and/or a humidity of the substrate and/or the pH value of the substrate. The ambient climate of the substrate may be characterised by an ambient temperature and/or an ambient humidity and/or the pH value of the substrate. Preferably, the term "ambient climate" refers to the or a medium directly surrounding or in direct contact with the substrate or a part of the substrate. For example, if the substrate (or a part thereof) is in contact with air, it is preferred that the ambient climate refers to the air, for example to the temperature and/or humidity of the air.

Preferably, the, one or more or all worm(s) provided in step i), where applicable, is/are a worm, preferably earthworm, with a fully developed clitellum.

Preferably, the term "cocoon", as used herein, refers to a fertilized ovum of a worm.

Preferably, the term "substrate" refers to a material comprising or consisting of decomposing vegetables or biodegradable food waste, one or more bedding material(s), soil, turf and combinations thereof. Preferably, the term "substrate" refers to a material comprising or consisting of soil, turf and combinations thereof.

Decomposing vegetables or bio-degradable food waste may be any organic material, which can be degraded by worms, insects or microorganisms.

Bedding material(s), which may be used as part of vermicompost may be selected from cardboard, paper, paper products, sawdust.

Typically, raising one or more worm(s), as described herein, produces vermicompost.

It is particularly advantageous in the method according to the invention that the amount of produced waste is strongly reduced, typically the method according to the invention provides a "zero-waste" approach, since the produced vermicompost can be used within the method according to the invention or can be sold as a highly ecological plant fertilizer.

The term "vermicompost", as used herein, refers to a mixture of decomposing vegetables or bio-degradable food waste, one or more bedding material(s) and vermicast. Preferably, the mixture further comprises worm feed, which is / was used for feeding the one or more worm(s) raised in the substrate.

The term "vermicast" describes the end-product of the breakdown of the decomposing vegetables or bio-degradable food waste, which is excreted by worms. Due to the breakdown process, vermicast typically contains a higher concentration of nutrients than the decomposing vegetables or bio-degradable food waste before the breakdown process.

The term "worm feed" preferably refers to or comprises one, two, three or more or all selected from peat, straw, hay, ground cereals, limestone, pulp, minerals, salts and sugars.

Preferably, the determining of at least one average property of the, one, two, three or more or all worm(s) in step iv) of the method according to the invention is performed by employing an imaging method that is capable of providing 2D and even more preferably, 3D information of the worms within the substrate. Suitable imaging methods may include using infrared light, x-rays, ionizing radiation, magnetic fields, magnetic field gradients, radio waves and combinations thereof, preferably for determining at least one average property of worms within the substrate. Thus, it is preferred that an imaging method is employed that is capable of providing one or more images of the worms present in the substrate. Each image may show a projection of the worms along one direction through the substrate. From the one or more images, 3D information about at least one average property of worms within the substrate may be determined.

Preferably, the determining of at least one average property of the, one, two, three or more or all worm(s) in step iv) of the method according to the invention is performed by a worm detection unit that is configured for determining the at least one average property of one or more worm(s) raised in the substrate. A worm detection unit may comprise an imaging scanner for producing one or more images of the worms present in the substrate. An imaging scanner may be a magnetic resonance imaging (MRI) scanner, a computed tomography (CT) scanner, a Positron emission tomography (PET) scanner, an ultra sound apparatus or combinations thereof. Preferably, a worm detection unit is configured for evaluating the images provided by the imaging scanner to automatically determine the at least one average property of the, one, two, three or more or all worm(s), e.g., using software. For example, the worm detection unit may include a worm detection artificial neural network that is trained for receiving images generated by an imaging scanner as input and for providing the at least one average property by evaluating the received images. The worm detection unit may be further configured to compare the provided at least one average property of the worms to a reference curve that represents an expected trend for at least one average property of the worms, e.g., as a function of time, or ambient temperature or ambient humidity. The worm detection unit may be further configured to also provide how the determined at least one average property of the worms relates to the reference curve. Based the comparison of the at least one average property of the worms to reference curve it is possible to control the ambient climate by adjusting the at least one climate parameter to influence a condition or a future condition of the worms present in the substrate. Based on the comparison of the at least one average property of the worms to reference curve it is also possible to control the ambient climate of substrate used for raising worms at a different place or in the future.

After measuring the number, size and/or weight in step iv) of the method according to the invention, a measured number and/or size and/or weight is obtained. Preferably, the measured size or weight refers to the average size or weight of the measured worms. Preferably, the measured size refers to the average length of the measured worms. Preferably, the size of the one or more worms is measured automatically, e.g., using a worm size determination unit.

Preferably, the automatic control in step v) of the method according to the invention is performed by an ambient climate control unit that is configured for controlling the ambient climate of the substrate, e.g., by controlling a temperature adjustment element and/or a humidity adjustment element and/or a pH adjustment element to provide a determined ambient climate. For example, the ambient climate control unit may provide a temperature control signal to control a temperature adjustment element to increase or decrease the current ambient temperature to reach a determined ambient temperature. Likewise, the ambient climate control unit may provide a humidity control signal to control a humidity adjustment element to increase or decrease the current ambient humidity to reach a determined ambient humidity. Likewise, the ambient climate control unit may provide a pH control signal to control a pH adjustment element to increase or decrease the current pH value of the substrate to reach a determined pH value.

Preferably, the temperature and/or the humidity of the substrate is adjusted by adjusting the temperature and/or the humidity of the ambient climate of the substrate. For example, the ambient climate control unit may be or may comprise a climate system that is configured for controlling the ambient climate, e.g., an ambient temperature and/or an ambient humidity of the air surrounding the substrate. Thus, the temperature and/or the humidity of the substrate will adapt to the temperature and/or humidity of the surrounding air.

Preferably, the pH value can be adjusted by adding one or more pH modifiers, preferably one or more acidifier(s) and/or one or more alkalizer(s), or a composition comprising one or more pH modifiers, preferably one or more acidifier(s) and/or one or more alkalizer(s).

It was found that the growth and breeding of worms can be influenced by the temperature and/or humidity of the substrate. The highest growth of worms was found to be achieved at a temperature in the range of from 12.5 to 20 °C, preferably 13 to 18 °C and a humidity of the substrate in the range of from 60 to 80 %.

Thus, for increasing the growth of the worms, the temperature and/or humidity can be set or approximated to these conditions. Likewise, for reducing the growth of the worms, i.e. for avoiding excessive production, the temperature and/or humidity may be reduced, preferably below the above temperature and/or humidity.

Preferably, the collecting in step iv) of the method according to the invention comprises or consists of one or more method(s) selected from the group consisting of sieving, manual collection and collection with a robot picking arm. For sieving, a drum screen may be employed.

The term "one or more method(s)", as used in this context, describes that the collection method may also comprise more than one different collection method, i.e. a combination of several methods, which are applied subsequently or simultaneously, wherein one or more of the several method(s) may also be applied more than once in the collecting step.

Furthermore, for providing a feed composition, transport substrate may be added to the one or more worm(s) collected in step vi) of the method according to the invention. Preferably, the transport substrate is added before storing the collected worm(s), as described below. Preferably, the transport substrate is added after storing the collected worm(s), as described below.

Preferably, the transport substrate, as described herein, is a substrate selected from the group consisting of soil, peat, cellulose and mixtures thereof.

Preferably, the term "essentially consisting of", as used herein, refers to a partially closed enumeration and designates composition which apart from the named components only have such further components, which do not materially alter the character of the composition according to the invention.

Preferably, the method according to the invention further comprises the step
- drying the one or more worm(s) collected in step vi) to obtain a dried feed composition.

The term "drying", as used herein, particularly in the method according to the invention, refers to a step, which reduces the water content of an object or a mixture. Preferably, the method for drying comprises or consists of one or more method(s) selected from the group consisting of heat drying, drying with a stream of nitrogen, sun drying, hot air drying, combined air and heat pump drying, oven drying, vacuum oven drying, freeze drying, belt drying and vacuum belt drying.

The term "one or more method(s)", as used in this context, describes that the drying method may also comprise more than one different drying method, i.e. a combination of several methods, which are applied subsequently, wherein one or more of the several method(s) may also be applied more than once in the drying step.

Preferably, the feed composition according to the invention substantially consists of the one or more worm(s) as described herein, wherein only residual amounts of vermicompost, as described herein, particularly preferably only residual amounts of components of vermicompost, as described herein, are present in the composition.

Further preferably, the amount of the one or more worm(s) in the feed composition is in a range of from 40 to 99 wt.-%, preferably in a range of from 45 to 95 wt.-%, particularly preferably in a range of from 50 to 92.5 wt.-%, further preferably in a range of from 55 to 90 wt.-%, preferably in a range of from 60 to 85 wt.-%, preferably in a range of from 65 to 80 wt.-%, preferably in a range of from 70 to 75 wt.-%, based on the total dry weight of the feed composition. Preferably, the further amount of the composition consists of or substantially consists of vermicompost as described herein.

Further preferably, the amount of the one or more worm(s) in the feed composition is in a range of from 0.5 to 50 wt.-%, preferably in a range of from 1 to 45 wt.-%, particularly preferably in a range of from 2 to 40 wt.-%, further preferably in a range of from 2.5 to 35 wt.-%, preferably in a range of from 3 to 30 wt.-%, preferably in a range of from 4 to 25 wt.-%, preferably in a range of from 5 to 20 wt.-%, based on the total wet weight of the feed composition. Preferably, the further amount of the composition consists of or substantially consists of vermicompost as described herein.

Preferably, the feed composition according to the invention substantially consists of the one or more worm(s) as described herein, wherein only residual amounts of one or more transport substrate(s), as described herein, are present in the composition.

Further preferably, the amount of the one or more worm(s) in the feed composition is in a range of from 40 to 99 wt.-%, preferably in a range of from 45 to 95 wt.-%, particularly preferably in a range of from 50 to 92.5 wt.-%, further preferably in a range of from 55 to 90 wt.-%, preferably in a range of from 60 to 85 wt.-%, preferably in a range of from 65 to 80 wt.-%, preferably in a range of from 70 to 75 wt.-%, based on the total dry weight of the feed composition. Preferably, the further amount of the composition consists of or substantially consists of one or more transport substrate(s), as described herein.

Further preferably, the amount of the one or more worm(s) in the feed composition is in a range of from 0.5 to 50 wt.-%, preferably in a range of from 1 to 45 wt.-%, particularly preferably in a range of from 2 to 40 wt.-%, further preferably in a range of from 2.5 to 35 wt.-%, preferably in a range of from 3 to 30 wt.-%, preferably in a range of from 4 to 25 wt.-%, preferably in a range of from 5 to 20 wt.-%, based on the total wet weight of the feed composition. Preferably, the further amount of the composition consists of or substantially consists of one or more transport substrate(s), as described herein.

Preferably, the feed composition according to the invention substantially consists of the one or more worm(s) as described herein, wherein only residual amounts of one or more transport substrate(s), as described herein, and of vermicompost, particularly preferably only residual amounts of components of vermicompost are present in the composition.

Further preferably, the amount of the one or more worm(s) in the feed composition is in a range of from 40 to 99 wt.-%, preferably in a range of from 45 to 95 wt.-%, particularly preferably in a range of from 50 to 92.5 wt.-%, further preferably in a range of from 55 to 90 wt.-%, preferably in a range of from 60 to 85 wt.-%, preferably in a range of from 65 to 80 wt.-%, preferably in a range of from 70 to 75 wt.-%, based on the total dry weight of the feed composition. Preferably, the further amount of the composition consists of or substantially consists of one or more transport substrate(s), as described herein, and vermicompost, as described herein.

Further preferably, the amount of the one or more worm(s) in the feed composition is in a range of from 0.5 to 50 wt.-%, preferably in a range of from 1 to 45 wt.-%, particularly preferably in a range of from 2 to 40 wt.-%, further preferably in a range of from 2.5 to 35 wt.-%, preferably in a range of from 3 to 30 wt.-%, preferably in a range of from 4 to 25 wt.-%, preferably in a range of from 5 to 20 wt.-%, based on the total wet weight of the feed composition. Preferably, the further amount of the composition consists of or substantially consists of one or more transport substrate(s), as described herein, and vermicompost, as described herein.

Preferably, the dry matter content of the feed composition according to the invention is at least 15 wt.-%, preferably at least 17.5 wt.-%, preferably at least 20 wt.-%, preferably at least 25 wt.-%, preferably at least 30 wt.-%, preferably at least 35 wt.-%, preferably at least 37.5 wt.-%, preferably at least 40 wt.-%, preferably at least 45 wt.-%, preferably at least 50 wt.-%, preferably at least 55 wt.-%, preferably at least 57.5 wt.-%, preferably at least 60 wt.-%, preferably at least 65 wt.-%, preferably at least 70 wt.-%, preferably at least 75 wt.-%, preferably at least 77.5 wt.-%, preferably at least 80 wt.-%, preferably at least 85 wt.-%, preferably at least 87.5 wt.-%, preferably at least 90 wt.-%, preferably at least 92.5 wt.-%, preferably at least 95 wt.-%, preferably at least 97.5 wt.-%, based on the total weight of the feed composition.

Preferably, the dry matter content of the feed composition according to the invention is in a range of from 10 to 30 wt.-%, preferably in a range of from 15 to 25 wt.-%, particularly preferably in a range of from 17.5 to 22.5 wt.-%, based on the total weight of the feed composition, and the amount of crude protein of the feed composition according to the invention is preferably at least 10 wt.-%, based on the total weight of the feed composition.

Preferably, the dry matter content of the feed composition according to the invention is in a range of from 30 to 50 wt.-%, preferably in a range of from 35 to 45 wt.-%, particularly preferably in a range of from 37.5 to 42.5 wt.-%, based on the total weight of the feed composition, and the amount of crude protein of the feed composition according to the invention is preferably at least 20 wt.-%, based on the total weight of the feed composition.

Preferably, the dry matter content of the feed composition according to the invention is in a range of from 50 to 70 wt.-%, preferably in a range of from 55 to 65 wt.-%, particularly preferably in a range of from 57.5 to 62.5 wt.-%, based on the total weight of the feed composition, and the amount of crude protein of the feed composition according to the invention is preferably at least 32.5 wt.-%, based on the total weight of the feed composition.

Preferably, the dry matter content of the feed composition according to the invention is in a range of from 70 to 90 wt.-%, preferably in a range of from 75 to 85 wt.-%, particularly preferably in a range of from 77.5 to 82.5 wt.-%, based on the total weight of the feed composition, and the amount of crude protein of the feed composition according to the invention is preferably at least 45 wt.-%, based on the total weight of the feed composition.

Preferably, the dry matter content of the feed composition according to the invention is in a range of from 80 to 97.5 wt.-%, preferably in a range of from 85 to 95 wt.-%, particularly preferably in a range of from 87.5 to 92.5 wt.-%, based on the total weight of the feed composition, and the amount of crude protein of the feed composition according to the invention is preferably at least 50 wt.-%, based on the total weight of the feed composition.

Preferably, the dry matter content of the feed composition according to the invention is in a range of from 85 to 99.9 wt.-%, preferably in a range of from 90 to 98 wt.-%, particularly preferably in a range of from 92.5 to 97.5 wt.-%, based on the total weight of the feed composition, and the amount of crude protein of the feed composition according to the invention is preferably at least 55 wt.-%, based on the total weight of the feed composition.

It is preferred that the feed composition according to the invention is a dried composition, preferably wherein the dried composition has a water content of at most 80 wt.-%, preferably at most 70 wt.-%, preferably at most 60 wt.-%, preferably at most 50 wt.-%, preferably at most 40 wt.-%, preferably at most 30 wt.-%, preferably at most 25 wt.-%, preferably of at most 20 wt.-%, preferably at most 15 wt.-%, preferably at most 12.5 wt.-%, preferably of at most 10 wt.-%, preferably at most 9 wt.-%, preferably at most 8 wt.-%, preferably at most 7 wt.-%, preferably at most 6 wt.-%, preferably of at most 5 wt.-%, based on the total weight of the dried composition.

Preferably, the water content, as described herein, is determined by Loss-on-Drying (LOD) method determined e.g. by a dry-mass balance or thermogravimetry or by Karl Fischer Titration. Particularly preferably, the water content is determined as described in EU Commission Regulation 152/2009 "Laying down the methods of sampling and analysis of the official control of feed"; Annex III: "Methods of analysis to control the composition of feed materials and compound feed" - A. Determination of moisture.

Furthermore, the feed composition according to the invention may contain one or more additive(s), for example such, which are typically included in feed provided for feeding particular animals, as described herein. The term "additive" refers to a substance in a feed composition, which is typically included in animal feed (such as feed provided for feeding pigs or piglets, or chickens or chicklets). Such additive(s) may be selected from the group consisting of corn, corn cob mix (CCM), soybeans, soybean meal, soy protein concentrates, wheat, barley, rye, triticale, starch, sugars, amino acids (particularly lysine and/or methionine), and dairy based feed products.

Preferably, the term "adjusting at least one climate parameter to control an ambient climate of the substrate in dependence on the determined average property of worms in the substrate " in step v) of the method according to the invention is to be understood such that, e.g., the temperature of the substrate and/or the humidity of the substrate and/or the pH value of the substrate is automatically controlled in case, in the measurement in step iv), it is found that the at least one average property, e.g., a number of worms in the substrate and/or an average size of the, one, two, three or more or all worm(s) in the substrate and/or an average weight of the, one, two, three or more or all worm(s) in the substrate has/have exceeded or, respectively, is below a predetermined value or range. In this way, the growth of the worms can be influenced in such a way that a particular output quantity (i.e. amount of worms produced) can be obtained at a given time. Thus, a sufficient output quantity is obtained, whereas no excess production of worms has occurred, which would represent a waste of energy and material.

The predetermined value or range is preferably determined based on the required output quantity (i.e. amount of worms produced) and the time, preferably remaining time, for producing said output quantity. Thus, the predetermined value or range may vary depending on the time point of measurement, i.e. on the remaining time for producing the output quantity at the time point of measurement.

Preferably, the term "amount of worms", as used herein, refers to the number, the size and/or the weight of the worms, preferably wherein the size and/or the weight refer to the average size and/or weight of a plurality of worms.

In step v), the "adjusting at least one climate parameter to control an ambient climate of the substrate" is preferably accomplished without a human intervention step. In particular, step v) may be implemented using software and hardware that is configured for automatically controlling an ambient climate, e.g., an ambient temperature of the substrate and/or an ambient humidity of the substrate in dependence on the determined at least one average property of the, one, two, three or more or all worm(s) in the substrate. For example, a temperature control signal may be generated and provided that is used in step v) to control hardware to increase or decrease the current ambient temperature to a determined ambient temperature of the substrate and/or a humidity control signal may be generated and provided that is used to increase or decrease a current ambient humidity of the substrate to a determined ambient humidity. A temperature control signal and/or a humidity control signal may be generated based on, e.g., a provided current ambient temperature and/or a provided current ambient humidity and a determined average property of the, one, two, three or more or all worm(s) in the substrate. For example, a measured number of worms in the substrate and/or an average size of the, one, two, three or more or all worm(s) in the substrate and/or an average weight of the, one, two, three or more or all worm(s) in the substrate may be analysed to determine whether according to the measured number of worms in the substrate and/or average size of the, one, two, three or more or all worm(s) in the substrate and/or average weight of the, one, two, three or more or all worm(s) in the substrate, the current ambient temperature of the substrate and/or the current ambient humidity of the substrate should be increased or decreased to a determined ambient temperature or determined ambient humidity, respectively, or left unchanged. Based on this determination, a corresponding temperature control signal and/or a humidity control signal may be generated to automatically control hardware, e.g. a heating element to provide the determined ambient temperature that may be lower, equal or larger than the current ambient temperature and/or, e.g., an automatic sprinkler system to provide the determined ambient humidity that may be lower, equal or larger than the current ambient humidity.

Hardware to increase or decrease the ambient temperature may be a heating element that is configured to convert electrical energy into heat. A heating element may be a metallic resistance heating elements, e.g., comprising a heating wire or a heating ribbon. A heating element may also be a ceramic heating element or a semiconductor heating element.

As described above, the growth of the worms can be increased or decreased by setting or approximating the ambient temperature and/or ambient humidity to the conditions with the highest growth or by reducing the ambient temperature and/or ambient humidity, preferably below the ambient temperature and/or ambient humidity with the highest growth as described above.

It is thus preferred in the method according to the invention that in step v), the temperature of the substrate is controlled to decrease to a determined temperature in the range of from 12 to 7.5 °C, preferably of from 9 to 11 °C.

Additionally or alternatively, it is thus preferred in the method according to the invention that in step v), the temperature of the substrate is controlled to decrease by at least 3 °C, preferably at least 4 °C, preferably at least 5 °C.

It is thus preferred that, additionally or alternatively, in step v), the humidity of the substrate is controlled to decrease by at least 5 %, preferably at least 7.5 %, preferably at least 10 %, preferably at least 15 %, preferably at least 20 %, preferably at least 25 %, based on 100 %.

Preferably, the terms "ambient humidity" and "humidity of the substrate", as used herein, refers to the water content of the medium, preferably the air, surrounding the substrate or, respectively, the substrate itself, respectively. Particularly preferably, the term "humidity of the substrate", as used herein, refers to the percentage weight of the water contained in the substrate, measured as the water content as described herein, based on the total weight of the substrate.

Further preferably, the temperature and/or the humidity is only decreased in a portion of the substrate. In this case, the term "humidity of the substrate", as used herein, refers to the water content of said portion of the substrate. Particularly preferably in this case, the term "humidity of the substrate", as used herein, refers to the percentage weight of the water contained in said portion of the substrate, measured as the water content as described herein, based on the total weight of said portion of the substrate.

Preferably, the term "decreasing the humidity by at least 5 %, based on 100%", as used herein, is to be understood such that the basis for calculating the decrease is 100 %, i.e. in case the humidity of e.g. 70 % is reduced by 5 %, based on 100 %, the humidity is reduced to 65 %. The same applies accordingly to changes in the humidity by different percentages.

It is thus preferred in the method according to the invention that in step v), the temperature of a portion of the substrate is controlled to decrease to a determined temperature in the range of from 12 to 7.5 °C, preferably of from 9 to 11 °C.

Additionally or alternatively, it is thus preferred in the method according to the invention that in step v), the temperature of a portion of the substrate is controlled to decrease by at least 4 °C, preferably at least 5 °C.

Additionally or alternatively, it is thus preferred that, additionally or alternatively, in step v), the humidity of a portion of the substrate is controlled to decrease by at least 5 %, preferably at least 7.5 %, preferably at least 10 %, preferably at least 15 %, preferably at least 20 %, preferably at least 25 %, based on 100 %.

Furthermore, it is preferred in the method according to the invention that providing one or more cocoon(s) is achieved by raising and feeding two or more worms and separating the worm cocoons from the raised worms.

Preferably, the method for separating the worm cocoons from the raised worms comprises or consists of one or more method(s) selected from the group consisting of sieving, manual separation and separation with a robot picking arm.

The term "one or more method(s)", as used in this context, describes that the separation method may also comprise more than one different separation method, i.e. a combination of several methods, which are applied subsequently or simultaneously, wherein one or more of the several method(s) may also be applied more than once in the separation step.

As described above, it has been found that the growth of worms is particularly high at a temperature in the range of from 12.5 to 20 °C, preferably of from 13 to 18 °C, and/or at a humidity in the range of from 60 to 80 %, based on 100 %.

Thus, it is preferred in the method according to the invention that in step iii), the temperature of the substrate is controlled to be in the range of from 12.5 to 20 °C, preferably of from 13 to 18 °C.

It is preferred that, additionally or alternatively, in step iii), the humidity of the substrate is controlled to be in the range of from 55 to 85 %, preferably in the range of from 60 to 80 %, based on 100 %.

Furthermore, it was found that with the method according to the invention, worms can be produced, which are free of specific pathogens. Since many or all steps in the method may be or, respectively, are performed automatically, the method may be performed in a closed system, where no direct human interaction is necessary or, respectively, possible. Thus, the risk of contaminating the one or more worm(s), the one or more cocoon(s), the substrate and/or the worm feed can be significantly reduced.

As described above, microorganisms such as *Enterotoxigenic Escherichia coli (ETEC),* preferably *Enterotoxigenic Escherichia coli (ETEC): F4 (K 88); Clostridium spp.,* preferably *Clostridium perfringens, Clostridium difficile; Brachyspira hyodysenteriae; Lawsonia intracellularis; Campylobacter spp.; Salmonella spp.,* preferably *S. typhimurium, S. coleraesuis; Brucella suis; Histomonas meleagridis; Streptococcus spp., Coliforms, Bacterioides, Eimeria* and *Avian Influenza-A virus* may cause a variety of diseases, which are harmful to animals, particularly animals raised for slaughter.

It is thus preferred that the one or more worm(s) and/or the one or more cocoon(s) provided in step i), and/or the substrate, and/or the worm feed,
preferably the one or more worm(s) and the one or more cocoon(s) provided in step i), and the substrate, and the worm feed,
is/are free of one, two, three or more or all pathogens selected from the group consisting of *Enterotoxigenic Escherichia coli (ETEC),* preferably *Enterotoxigenic Escherichia coli (ETEC): F4 (K 88); Clostridium spp.,* preferably *Clostridium perfringens, Clostridium difficile; Brachyspira hyodysenteriae; Lawsonia intracellularis; Campylobacter spp.; Salmonella spp.,* preferably *S. typhimurium, S. coleraesuis; Brucella suis; Histomonas meleagridis; Streptococcus spp., Coliforms, Bacterioides, Eimeria* and *Avian Influenza-A virus,*
preferably is/are free of all pathogens selected from the group consisting of *Enterotoxigenic Escherichia coli (ETEC),* preferably *Enterotoxigenic Escherichia coli (ETEC): F4 (K 88); Clostridium spp.,* preferably *Clostridium perfringens, Clostridium difficile; Brachyspira hyodysenteriae; Lawsonia intracellularis; Campylobacter spp.; Salmonella spp.,* preferably *S. typhimurium, S. coleraesuis;* and *Brucella suis,*
preferably is/are free of all pathogens selected from the group consisting of *Campylobacter spp.; Salmonella spp.,* preferably *S. typhimurium, S. coleraesuis;* and *Histomonas meleagridis.*

Preferably, the term "free of a pathogen" or "pathogen free", as used herein, e.g., in view of a worm, a cocoon, or of an object, is to be understood such that if from 100 g of the worm or of the object 25 g are analysed by PCR, preferably using 18S rRNA as internal control and/or a 16S rRNA based bacterial community analysis, no detection of the respective pathogen is observed. Moreover, it is preferred that the analysis by PCR is or comprises a step of performing RT-PCR. Typically, the testing is repeated at regular, defined intervals to maintain the status of an animal being "free of a pathogen", as described herein.

The term "object" as used herein may comprise, for example, the feed composition, vermicompost, the vermicast, the substrate or the worm feed.

Further preferably, the term "free of *Salmonella",* as used herein, includes that the 25 g of the worm or object are analysed according to DIN EN ISO 6579-1, 2017-07 and that no species of Salmonella is detected.

Further preferably, the term "free of *Campylobacter",* as used herein, includes that the 25 g of the worm or object are analysed according to DIN ISO 21528-2, 2017-09 and the total amount of species of Campylobacter detected is 1.000.000 KbE/g or less.

Further preferably, the term "free of *Clostridium",* as used herein, includes that the 25 g of the worm or object are analysed according to DIN ISO 15213-1.

Further preferably, the term "free of *Clostridium perfringens",* as used herein, includes that the 25 g of the worm or object are analysed according to DIN EN ISO 7937 (Lebens- u. Futtermittel; "Techn. Regel" BVL F 0105 (Futtermittel).

Further preferably, the term "free of *Brucella suis",* as used herein, includes that the 25 g of the worm or object are analysed according to NF U47-105.

Preferably, the one or more worm(s) and/or the one or more cocoon(s) provided in step i), and/or the substrate, and/or the worm feed,
preferably the one or more worm(s) and the one or more cocoon(s) provided in step i), and the substrate, and the worm feed,
is/are at least free of *Enterotoxigenic Escherichia coli (ETEC),* preferably *Enterotoxigenic Escherichia coli (ETEC): F4 (K 88).*

Preferably, the one or more worm(s) and/or the one or more cocoon(s) provided in step i), and/or the substrate, and/or the worm feed,
preferably the one or more worm(s) and the one or more cocoon(s) provided in step i), and the substrate, and the worm feed,
is/are at least free of *Clostridium spp.,* preferably *Clostridium perfringens,* preferably Clos*tridium difficile.*

Preferably, the one or more worm(s) and/or the one or more cocoon(s) provided in step i), and/or the substrate, and/or the worm feed,
preferably the one or more worm(s) and the one or more cocoon(s) provided in step i), and the substrate, and the worm feed,
is/are at least free of *Brachyspira hyodysenteriae.*

Preferably, the one or more worm(s) and/or the one or more cocoon(s) provided in step i), and/or the substrate, and/or the worm feed,
preferably the one or more worm(s) and the one or more cocoon(s) provided in step i), and the substrate, and the worm feed,
is/are at least free of *Lawsonia intracellularis.*

Preferably, the one or more worm(s) and/or the one or more cocoon(s) provided in step i), and/or the substrate, and/or the worm feed,
preferably the one or more worm(s) and the one or more cocoon(s) provided in step i), and the substrate, and the worm feed,
is/are at least free of *Campylobacter spp..*

Preferably, the one or more worm(s) and/or the one or more cocoon(s) provided in step i), and/or the substrate, and/or the worm feed,
preferably the one or more worm(s) and the one or more cocoon(s) provided in step i), and the substrate, and the worm feed,
is/are at least free of *Salmonella spp.,* preferably *S. typhimurium,* preferably *S. coleraesuis.*

Preferably, the one or more worm(s) and/or the one or more cocoon(s) provided in step i), and/or the substrate, and/or the worm feed,
preferably the one or more worm(s) and the one or more cocoon(s) provided in step i), and the substrate, and the worm feed,
is/are at least free of *Brucella suis.*

Preferably, the one or more worm(s) and/or the one or more cocoon(s) provided in step i), and/or the substrate, and/or the worm feed,
preferably the one or more worm(s) and the one or more cocoon(s) provided in step i), and the substrate, and the worm feed,
is/are at least free of *Histomonas meleagridis.*

Preferably, the one or more worm(s) and/or the one or more cocoon(s) provided in step i), and/or the substrate, and/or the worm feed,
preferably the one or more worm(s) and the one or more cocoon(s) provided in step i), and the substrate, and the worm feed,
is/are at least free of *Streptococcus spp..*

Preferably, the one or more worm(s) and/or the one or more cocoon(s) provided in step i), and/or the substrate, and/or the worm feed,
preferably the one or more worm(s) and the one or more cocoon(s) provided in step i), and the substrate, and the worm feed,
is/are at least free of *Coliforms.*

Preferably, the one or more worm(s) and/or the one or more cocoon(s) provided in step i), and/or the substrate, and/or the worm feed,
preferably the one or more worm(s) and the one or more cocoon(s) provided in step i), and the substrate, and the worm feed,
is/are at least free of *Bacterioides.*

Preferably, the one or more worm(s) and/or the one or more cocoon(s) provided in step i), and/or the substrate, and/or the worm feed,
preferably the one or more worm(s) and the one or more cocoon(s) provided in step i), and the substrate, and the worm feed,
is/are at least free of *Eimeria.*

Preferably, the one or more worm(s) and/or the one or more cocoon(s) provided in step i), and/or the substrate, and/or the worm feed,
preferably the one or more worm(s) and the one or more cocoon(s) provided in step i), and the substrate, and the worm feed,
is/are at least free of *Avian Influenza-A virus.*

Advantageously, the method for producing a feed composition according to the invention is performed such that during its steps, a contamination with one or more pathogen(s), of which the one or more worm(s) and/or the one or more cocoon(s) provided in step i), and/or the substrate, and/or the worm feed are free of, is prevented.

Advantageously, the method for producing a feed composition according to the invention is performed such that during its steps, a contamination with one or more pathogen(s), of which the one or more worm(s) and the one or more cocoon(s) provided in step i), and the substrate, and the worm feed are free of, is prevented.

For example, in case the one or more worm(s) and/or the one or more cocoon(s) provided in step i), and/or the substrate, and/or the worm feed are free of *Clostridium perfringens,* a contamination with *Clostridium perfringens* during the method according to the invention is prevented.

It is further advantageous in the method according to the invention that the worms may be stored after collection, preferably at a low temperature. It is thus preferred in the method according to the invention that the method further comprises the step: vii) storing the collected worm(s), preferably, at a temperature in the range of from 5 to 12.5 °C, preferably 7.5 to 11 °C.

The present invention further relates to a system for producing a feed composition, comprising, essentially consisting of or consisting of one or more worm(s). The system comprises:
a) a container that is configured for enabling a control of an ambient climate within the container and for accommodating one or more boxes for raising worms, the one or more boxes being exposed to the ambient climate within the container,
b) one or more boxes that are configured for accommodating a substrate that is suitable for raising worm(s) and/or worm(s) hatched from cocoon(s),
c) an ambient climate control unit that is configured for adjusting at least one climate parameter to control the ambient climate within the container,
d) a worm detection unit that is configured for automatically determining at least one average property of the, one, two, three or more or all worm(s) present in the substrate, and
e) an ambient climate determination unit that is configured for determining at least one climate parameter in dependence on the determined average property of worms in the substrate for controlling the ambient climate in the container.

The system can be employed for conducting the steps of the method for producing a feed composition, comprising, essentially consisting of or consisting of one or more worm(s) as described above.

The system enables a pathogen-free (SPF) cultivation of live feed, i.e., of worms on an industrial scale. Moreover, the system enables a standardized and all year production of live feed. In particular, the system can be operated fully automated. Thereby, it is possible to achieve with the system a freely scalable worm production, e.g., with centralized, cloud-based production planning and controlling systems. Moreover, it is possible to achieve an SPF (specific pathogen free) certifiable and quality-controlled worm production for use of worms in the human food chain. Advantageously, with the system it is possible to provide a minimally invasive production using, e.g., non-invasive sensor and picture technology which may reduce or avoid direct human interaction (which may e.g. provide contamination with microorganisms), and avoiding light, and vibrations or mechanical selection processes where possible. With the system it is also possible to achieve a zero-waste production, since every production output can be used or re-used: i.e., worms, water, and soil, wherein soil may convert to highly ecological fertilizer, called vermicompost. Moreover, the system allows a carbon footprint reduction, e.g., by a sustainable energy supply of production facilities, and decentralized facilities close to the farming clusters/hotspots at different places, so logistics/transportation can be reduced to a minimum.

In particular, with the system, at least some of the following advantages can be realized: an output quantity over time or up to a predetermined time is predictable and controllable, worms can be produced all over the year, and worm production at industrial scale is made possible. Furthermore, it is possible to fulfil the requirements (specific pathogen-free) to produce live feed to be used in the human food chain which is required for a usage of worms as live feed. Due to the full automation of the live feed production process which is now possible using the system, worm production can be accomplished comparatively efficient and reliable.

With the system, it is possible to provide a controlled micro climate within the container as closed and self-contained system. In particular, with the system, it is possible to provide a micro-climate within the container that is substantially decoupled from the surrounding climate outside the container. Thereby, the system can be operated with combatively lower energy consumption, i.e., comparatively energy-efficient.

Preferably, the ambient climate control unit comprises a temperature adjustment element that is configured for increasing or decreasing an ambient temperature in the container to a determined ambient temperature, and/or a humidity adjustment element that is configured for increasing or decreasing an ambient humidity in the container to a determined ambient humidity and/or a pH value adjustment element that is configured for increasing or decreasing a pH value of the substrate.

Preferably, the temperature adjustment element is or comprises a heater or a heating element that is arranged and configured to increase or decrease a temperature accommodated in the container. Particularly preferably, the temperature adjustment element comprises a temperature sensor to measure the current temperature of the substrate. Based on the determined current temperature, the control unit may control the temperature adjustment element to increase or decrease the current temperature to a determined temperature to increase or decrease the growth rate of the worms. Whether the determined temperature of the substrate has been reached can likewise be measured with the temperature sensor. It is thus possible to establish a feedback loop that includes measuring the current ambient temperature and to increase or decrease the current ambient temperature until the measured current ambient temperature corresponds to the determined ambient temperature.

Preferably, the humidity adjustment element is or comprises a sprinkler system that is configured to apply water, preferably vaporized water, to increase the humidity within the container.

Preferably, the container is configured such that the ambient climate within the container can be controlled substantially independently from the climate outside the container.

Particularly preferably, the humidity adjustment element comprises a humidity sensor to measure the current humidity of the substrate. As describe for the temperature, the ambient climate control unit may also control the humidity adjustment element to increase or decrease the current humidity to reach the determined ambient humidity. Whether the determined ambient humidity has been reached can be measured with the humidity sensor. Also with respect to the ambient humidity, it is thus possible to establish a feedback loop that includes measuring the current ambient humidity and to increase or decrease the current ambient humidity until the measured current ambient humidity corresponds to the determined ambient humidity as determined with the temperature and/or humidity determination unit.

It is preferred that the system according to the invention comprises a collector device that is configured for collecting one, two, three or more or all worm(s) from the substrate. The collector device may be or may include a picking arm that is configured for picking worms, e.g., from a collection site, and to transfer them to a storage site, e.g., a storage box.

Preferably the collector device is selected from the group consisting of a picking arm, or a drum screen or combinations thereof.

The system, preferably, relies on optical detection methods of worm size. For example, the system's worm detection unit may comprise an imaging scanner that is configured for generating one or more images, preferably, from several different directions, of the worms present in the substrate. The imaging scanner can be a CT arm, an MRI scanner or the like.

Preferably, the imaging scanner is configured to provide image data indicative of worms present in the substrate. Preferably, the worm detection unit is configured to determine at least one average property of worms within the substrate in three-dimensional space.

With the worm detection unit, it is possible to automatically determine a size of one or more worm(s) raised in the substrate. Thereby, manually checking the size of one or more worm(s) raised in the substrate becomes obsolete.

Furthermore, it is preferred in the system according to the invention that the worm detection unit comprises a worm detection artificial neural network that is trained for determining at least one average property of the, one, two, three or more or all worm(s) present in the substrate. For example, for determining at least one average property of worms present in the substrate, the worm detection unit may comprise a worm detection artificial neural network that is trained for receiving age data indicative of worms present in the substrate as input and for determining the at least one average property of worms within the substrate as output. Training data for training the worm detection artificial neural network accordingly may represent images of worms present in a substrate and associated with each image one or more corresponding average properties of the, one, two, three or more or all worm(s) present in the respective substrates.

Furthermore, it is preferred that the ambient climate determination unit comprises an ambient climate artificial neural network that is configured to receive the determined at least one average property as input and that is trained for outputting the at least one climate parameter in dependence on the determined average property of worms in the substrate for controlling the ambient climate in the container. In particular, when employing the ambient climate artificial neural network, it is possible to train the ambient climate artificial neural network such that it provides a determined ambient temperature and/or a determined ambient humidity with which is suitable for enabling the worms to grow quickly. That is, the ambient climate artificial neural network can be trained to provide a determined ambient temperature of the substrate and/or a determined ambient humidity that is suitable according to the current average property for the worms to continue to grow relatively faster or, if preferred, to grow less fast. This is possible since the ambient climate artificial neural network can be trained to output for a respective determined average property of the worms a determined ambient temperature and/or a determined ambient humidity that are suitable to support a relatively faster or slower progress in worm growth. Thereby, the amount of worms that are ready for collection per time instance can be controlled, e.g., increased or decreased and the whole process of providing live feed can be made more efficient, e.g., also by avoiding overgrowth of worms.

Advantageously, the whole production process can be carried out with the system fully automated and controlled, e.g., by employing an ambient climate artificial neural network. The ambient climate artificial neural network may be used and trained to continuously improve the production process by using the collected data, e.g., for retraining the ambient climate artificial neural network. Training data for improvement may be gathered across several different production sites distributed over severely different places and fed into the ambient climate artificial neural network for retraining. Usage of an ambient climate artificial neural network thereby allows to control and continuously improve the production process also across several production sites that are distributed over different places.

It is further preferred that the system according to the invention comprises a training unit that is configured for training an ambient climate artificial neural network using training data and for providing the trained artificial neural network to the ambient climate determination unit.

The trained ambient climate artificial neural network and/or the trained worm detection artificial neural network can be a multi-scale neural network or a recurrent neural network (RNN) such as, but not limited to, a gated recurrent unit (GRU) recurrent neural network or a long short-term memory (LSTM) recurrent neural network. Alternatively, the ambient climate artificial neural network and/or the worm detection artificial neural network may be a convolutional neural network (CNN). Yet, it is generally preferred that the ambient climate artificial neural network and/or the worm detection artificial neural network of the system has a feed-forward topology.

The ambient climate neural artificial network may be trained using training data, e.g., representing at least one property of worms and corresponding climate parameters of an ambient climate in which these worms have been raised. Thereby, the ambient climate artificial network can learn a relation between certain average properties of worms and corresponding climate parameters associated with the average properties of the worms. For example, if the ambient climate artificial neural network is a feed-forward neural network such as a CNN, a backpropagation-algorithm may be applied for training the neural network. In case of a RNN, a gradient descent algorithm or a back-propagation-through-time algorithm may be employed for training purposes. As a result of the training, operating parameters for the neural network circuitry are generated such that when receiving image data indicative of the determined average property of the worms as input, the trained ambient climate artificial neural network outputs one or more corresponding climate parameters for controlling the ambient climate in a container in which worms are raised. The training data can be obtained prior to use of the system, i.e., as historical data. Training data may be generated while operating a system and subsequently used for training the ambient climate artificial neural network. For example, there may be several test runs of the system during which training data are generated and used for training the ambient climate artificial neural network. Furthermore, training data may be generated with a first system and used for training the ambient climate artificial neural network of a second system.

It is also possible to use image data and associated climate parameters, collected during operation of the system for retraining a trained ambient climate artificial neural network. For example, a copy of the presently used trained ambient climate artificial neural network may be retrained using training data generated while operating the system. The retrained ambient climate artificial neural network may replace the presently used trained ambient climate artificial neural network to further improve the efficiency of the whole system.

Training data may also be generated by several different systems that are operated in parallel, e.g., at different places. Thereby, it is possible to provide a comparatively large training data set that may contribute to improved training results of an ambient climate artificial neural networks. Thereby, too, the efficiency of the system can be further improved.

Suitable training data generally represent various different measured average properties of worms such as an average worm size or weight and associated with the measured worm size or weight determined climate parameters such as ambient temperature and/or ambient humidity that contribute a controlled growth of the worms.

The present invention further relates to a computer program for controlling an ambient climate in a container, e.g., by controlling an ambient temperature and/or ambient humidity and/or pH value of a substrate suitable for raising worm(s) and/or worm(s) hatched from cocoon(s), the computer program including instructions for
- receiving worm detection data indicative of a at least one average property of one or more worm(s),
- determining at least one climate parameter in dependence on the determined average property of worms in the substrate for controlling the ambient climate in the container, and
- providing control instructions for controlling the ambient climate in the container, when run on a computing device.

The computer program may be executed, for example, by the above described system's ambient climate determination unit and the control instructions for controlling the ambient climate in the container may be provided to the system's ambient climate control unit, e.g., for controlling the system's temperature adjustment element and/or humidity adjustment element and/or pH adjustment element. Preferably, the computer program according to the invention is or includes a trained ambient climate artificial neural network as described above.

Furthermore, the present invention relates to a non-transitory computer readable data medium storing the computer program according to the invention.

It shall be understood that the aspects described above, and specifically the method of claim 1, the system of claim 7 and the computer program of claim 14, have similar and/or identical preferred embodiments, in particular as defined in the dependent claims.

It shall be understood that a preferred embodiment of the invention can also be any combination of the dependent claims or above embodiments with the respective independent claim.

These and other aspects of the invention will be apparent from and elucidated with reference to the embodiments described hereinafter.

### BRIEF DESCRIPTION OF THE DRAWINGS

Fig. 1 shows a flowchart diagram representing a method for producing a feed composition, comprising, essentially consisting of or consisting of one or more worm(s); and
Fig. 2 shows a schematic representation of a system for producing a feed composition, comprising, essentially consisting of or consisting of one or more worm(s).

### DETAILED DESCRIPTION OF EMBODIMENTS

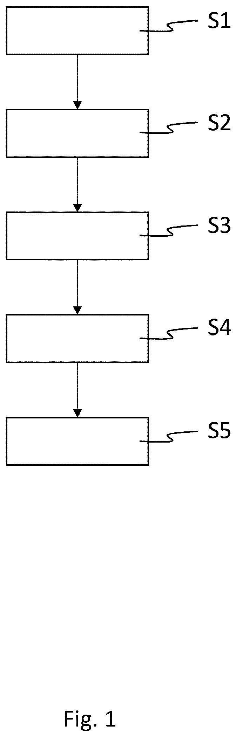

Figure 1 shows a flowchart diagram representing a method for producing a feed composition comprising one or more worms, wherein a particular amount of worms can be produced up to a determined time point (end point). In the method, initially, a plurality of cocoons of worms are put on a grid in a box above a substrate containing soil (step S1). The box is accommodated in a container in which a microclimate is established and controlled. The box is then exposed to the microclimate within the container. The microclimate can be defined in particular by an ambient temperature and an ambient humidity within the container. In particular, the microclimate within the container can be controlled substantially independently of the climate outside the container. In the container, a microclimate is automatically controlled such that in the box, worms hatch from the cocoons. Once the worms hatch, they move down wards into the substrate that is underneath the grid. In the substrate, the worms are raised, which includes providing worm feed to the worms present in and/or added to the substrate (step S2). The container is temperature and humidity controlled, e.g. via the surrounding air, such that it is possible to increase or decrease the temperature of the substrate and/or the humidity of the substrate in the box, thereby influencing the conditions for the worms to grow. Using worm detection, at least one average property of the, one, two, three or more or all worm(s) present in the substrate is determined. This may include determining a number and/or an average size and/or an average weight with a worm detection artificial neural network (step S3). The determined number of worms in the substrate and/or average size of the, one, two, three or more or all worm(s) in the substrate and/or average weight of the, one, two, three or more or all worm(s) in the substrate is provided as input to an ambient climate artificial neural network that is trained for determining based on the at least one average property of the, one, two, three or more or all worm(s) present in the substrate at least one climate parameter in dependence on the determined average property of worms, e.g. a substrate temperature and/or a substrate humidity and/or a substrate pH value that is associated with an increased or, respectively, decreased growth of the worms. The ambient temperature in the container is then automatically controlled to reach said substrate temperature and/or the humidity in the container is automatically controlled to reach said substrate humidity (step S4). Steps S3 and S4 may be repeated once or several times. Once the end point has been achieved, the worms are collected using a drum screen (step S5). Alternatively to a drum screen, a robot picking arm may be used or any further suitable method for collection can be used. The collected worms can then be packaged and moved, e.g., to a cold room for storage e.g. until shipment. Thereby the method allows producing a particular amount of worms up to a determined time point and in a fully automated manner, e.g., controlled by means of an ambient climate artificial neural network.

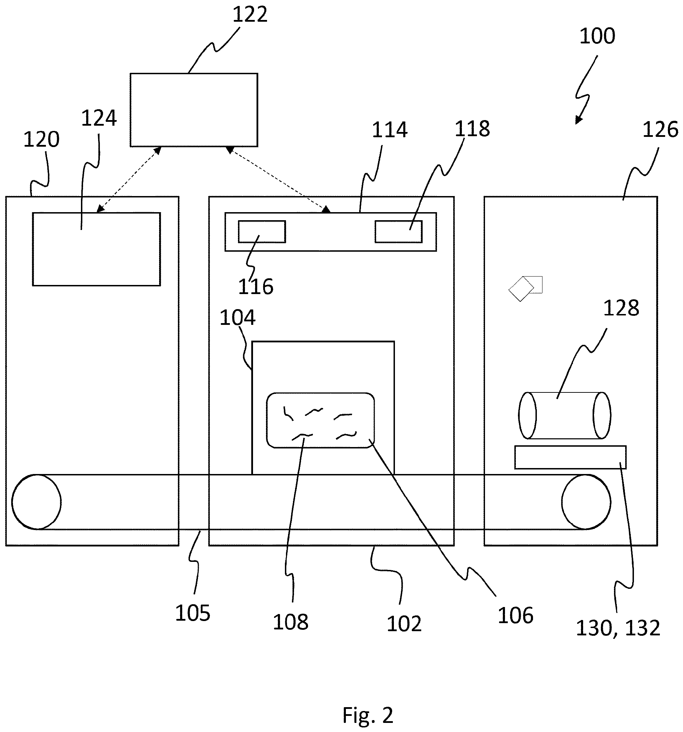

Figure 2 shows a schematic representation of a system 100 for producing a feed composition comprising worms. The system 100 can be used for conducting the method for producing a feed composition as described with reference to figure 1.

The system 100 comprises a container 102. In the container 102, there is at least one box 104 accommodating a substrate 106, e.g. made of or comprising soil, and containing severalworms 108. The worms 108 are hatched from cocoons.

To control the ambient climate, i.e., the microclimate, within the container 102, the system 100 comprises an ambient climate control unit 114. The ambient climate control unit 114 comprises a temperature adjustment element 116 that includes heating elements that are arranged and configured for increasing or decreasing an ambient temperature within the container 102. Moreover, the ambient climate control unit 114 includes a humidity adjustment element 118 that includes a sprinkler system that may apply vaporized water increase the ambient humidity in the container 102.

In addition, the system 100 includes a worm detection unit 120 that includes an imaging scanner 124, e.g., a CT arm or an MRI scanner that is configured to detect worms 108 within the substrate. To this end, the worm detection unit 120 includes a worm detection artificial neural network that is trained for automatically determining at least one average property, e.g., an amount of worms, e.g., an average weight or an average size, of the, one, two, three or more or all worm(s) present in the substrate. For determining at least one average property of the worms, the box 104 is transported e.g. via a roller conveyor 105 out of the container 102 and to the imaging scanner 124 of the worm detection unit 120. Having determined at least one average property of the worms, the box 104 is transported back into the container 102 with the roller conveyor 105.

The system 100 further includes an ambient climate determination unit 122 that is configured for determining at least one climate parameter, e.g., an ambient temperature and/or an ambient humidity, in dependence on the determined average property of worms in the substrate for controlling the ambient climate in the container. To this end, in operation, the ambient climate determination unit 122 receives the determined at least one average property of the worms from the warm detection unit 120 and determines based on the determined average property the at least one climate parameter. To this end, the ambient climate determination unit 122 includes an ambient climate artificial neural network that was trained for receiving the at least one average property of the, one, two, three or more or all worm(s) present in the substrate as input and for providing the at least one climate parameter in dependence on the determined average property. Based on the determined at least one climate parameter, the ambient climate control unit 120 may control the ambient climate in the container 102.

Using the roller conveyor 105, the box 104 with the worms 108 can be transported to a collector device 126 that includes a drum screen 128 that is used for collecting worms 108, e.g., that have reached a predetermined size. The drum screen 128 is located above a collection site 130 in form of a storage box 132 that can be used for storing the collected worms 108. The ready-to-sale worms can then be packaged and further moved to a cold room for storage until shipment.

Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality.

A single unit or device may fulfill the functions of several items recited in the claims. The mere fact that certain measures are recited in mutually different dependent claims does not indicate that a combination of these measures cannot be used to advantage.

Procedures like detecting an average property of the worm(s), and automatically controlling an ambient climate in the container, etc. performed by one or several units or devices can be performed by any other number of units or devices. These procedures can at least in part be implemented as program code means of a computer program and/or as dedicated hardware.

A computer program may be stored/distributed on a suitable medium, such as an optical storage medium or a solid-state medium, supplied together with or as part of other hardware, but may also be distributed in other forms, such as via the Internet or other wired or wireless telecommunication systems.

Any units described herein may be processing units that are part of a classical computing system. Processing units may include a general-purpose processor and may also include a field programmable gate array (FPGA), an application specific integrated circuit (ASIC), or any other specialized circuit. Any memory may be a physical system memory, which may be volatile, non-volatile, or some combination of the two. The term "memory" may include any computer-readable storage media such as a non-volatile mass storage. If the computing system is distributed, the processing and/or memory capability may be distributed as well. The computing system may include multiple structures as "executable components". The term "executable component" is a structure well understood in the field of computing as being a structure that can be software, hardware, or a combination thereof. For instance, when implemented in software, one of ordinary skill in the art would understand that the structure of an executable component may include software objects, routines, methods, and so forth, that may be executed on the computing system. This may include both an executable component in the heap of a computing system, or on computer-readable storage media. The structure of the executable component may exist on a computer-readable medium such that, when interpreted by one or more processors of a computing system, e.g., by a processor thread, the computing system is caused to perform a function. Such structure may be computer readable directly by the processors, for instance, as is the case if the executable component were binary, or it may be structured to be interpretable and/or compiled, for instance, whether in a single stage or in multiple stages, so as to generate such binary that is directly interpretable by the processors. In other instances, structures may be hard coded or hard wired logic gates, that are implemented exclusively or near-exclusively in hardware, such as within a field programmable gate array (FPGA), an application specific integrated circuit (ASIC), or any other specialized circuit. Accordingly, the term "executable component" is a term for a structure that is well understood by those of ordinary skill in the art of computing, whether implemented in software, hardware, or a combination. Any embodiments herein are described with reference to acts that are performed by one or more processing units of the computing system. If such acts are implemented in software, one or more processors direct the operation of the computing system in response to having executed computer-executable instructions that constitute an executable component. Computing system may also contain communication channels that allow the computing system to communicate with other computing systems over, for example, network. A "network" is defined as one or more data links that enable the transport of electronic data between computing systems and/or modules and/or other electronic devices. When information is transferred or provided over a network or another communications connection, for example, either hardwired, wireless, or a combination of hardwired or wireless, to a computing system, the computing system properly views the connection as a transmission medium. Transmission media can include a network and/or data links which can be used to carry desired program code means in the form of computer-executable instructions or data structures and which can be accessed by a general-purpose or special-purpose computing system or combinations. While not all computing systems require a user interface, in some embodiments, the computing system includes a user interface system for use in interfacing with a user. User interfaces act as input or output mechanism to users for instance via displays.

Those skilled in the art will appreciate that at least parts of the invention may be practiced in network computing environments with many types of computing system configurations, including, personal computers, desktop computers, laptop computers, message processors, hand-held devices, multi-processor systems, microprocessor-based or programmable consumer electronics, network PCs, minicomputers, main-frame computers, mobile telephones, PDAs, pagers, routers, switches, datacenters, wearables, such as glasses, and the like. The invention may also be practiced in distributed system environments where local and remote computing system, which are linked, for example, either by hardwired data links, wireless data links, or by a combination of hardwired and wireless data links, through a network, both perform tasks. In a distributed system environment, program modules may be located in both local and remote memory storage devices.

Those skilled in the art will also appreciate that at least parts of the invention may be practiced in a cloud computing environment. Cloud computing environments may be distributed, although this is not required. When distributed, cloud computing environments may be distributed internationally within an organization and/or have components possessed across multiple organizations. In this description and the following claims, "cloud computing" is defined as a model for enabling on-demand network access to a shared pool of configurable computing resources, e.g., networks, servers, storage, applications, and services. The definition of "cloud computing" is not limited to any of the other numerous advantages that can be obtained from such a model when deployed. The computing systems of the figures include various components or functional blocks that may implement the various embodiments disclosed herein as explained. The various components or functional blocks may be implemented on a local computing system or may be implemented on a distributed computing system that includes elements resident in the cloud or that implement aspects of cloud computing. The various components or functional blocks may be implemented as software, hardware, or a combination of software and hardware. The computing systems shown in the figures may include more or less than the components illustrated in the figures and some of the components may be combined as circumstances warrant.

Any reference signs in the claims should not be construed as limiting the scope.

## Claims

1. A method for producing a feed composition, comprising, essentially consisting of or consisting of one or more worm(s), the method comprising the steps:
i) providing one or more worm(s) and/or providing one or more cocoon(s) of one or more worm(s),
ii) placing the worm(s) and/or cocoon(s) provided in step i) on or within a substrate,
iii) raising the provided worm(s) and/or worm(s) hatched from the cocoon(s),
wherein raising includes providing worm feed to the one or more worm(s),
iv) automatically determining at least one average property of worms present in the substrate,
v) automatically adjusting at least one climate parameter to control an ambient climate of the substrate in dependence on the determined average property of worms in the substrate, and
vi) collecting the, one, two, three or more or all worm(s) to obtain the feed composition.

2. The method according to claim 1, wherein providing one or more cocoon(s) is achieved by raising and feeding two or more worms and separating the worm cocoons from the raised worms, preferably, by sieving or manually or with a robot picking arm.

3. The method according to any one of the preceding claims, wherein in step iii), the temperature of the substrate is controlled to be in the range of from 12.5 to 20 °C, preferably of from 13 to 18 °C, and/or
wherein in step iii), the humidity of the substrate is controlled to be in the range of from 60 to 80 %.

4. The method according to any one of the preceding claims, wherein in step v), the temperature of the substrate is controlled to decrease to a predetermined temperature in the range of from 12 to 7.5 °C, preferably of from 9 to 11 °C, and/or
wherein in step v), the temperature of the substrate is controlled to decrease by at least 3 °C, preferably at least 4 °C, preferably at least 5 °C, and/or
wherein in step v), the humidity of the substrate is controlled to decrease by at least 5 %, preferably at least 7.5 %, preferably at least 10 %, preferably at least 15 %, preferably at least 20 %, preferably at least 25 %, based on 100 %.

5. The method according to any one of the preceding claims, wherein the one or more worm(s) and/or the one or more cocoon(s) provided in step i), and/or the substrate, and/or the worm feed,
preferably the one or more worm(s) and the one or more cocoon(s) provided in step i), and the substrate, and the worm feed,
is/are free of one, two, three or more or all pathogens selected from the group consisting of *Enterotoxigenic Escherichia coli (ETEC),* preferably *Enterotoxigenic Escherichia coli (ETEC): F4 (K 88); Clostridium spp.,* preferably *Clostridium perfringens, Clostridium difficile; Brachyspira hyodysenteriae; Lawsonia intracellularis; Campylobacter spp.; Salmonella spp.,* preferably *S. typhimurium, S. coleraesuis; Brucella suis; Histomonas meleagridis; Streptococcus spp., Coliforms, Bacterioides, Eimeria* and *Avian Influenza-A virus,*
preferably is/are free of all pathogens selected from the group consisting of *Enterotoxigenic Escherichia coli (ETEC),* preferably *Enterotoxigenic Escherichia coli (ETEC): F4 (K 88); Clostridium spp.,* preferably *Clostridium perfringens, Clostridium difficile; Brachyspira hyodysenteriae; Lawsonia intracellularis; Campylobacter spp.; Salmonella spp.,* preferably *S. typhimurium, S. coleraesuis;* and *Brucella suis,*
preferably is/are free of all pathogens selected from the group consisting of *Campylobacter spp.; Salmonella spp.,* preferably *S. typhimurium, S. coleraesuis;* and *Histomonas meleagridis.*

6. The method according to any one of the preceding claims, wherein the method further comprises the step:
vii) storing the collected worm(s), preferably, at a temperature in the range of from 5 to 12.5 °C, preferably 7.5 to 11 °C.

7. A system for producing a feed composition, comprising, essentially consisting of or consisting of one or more worm(s), preferably, by the method according to any one of the preceding claims, wherein the system comprises:
a) a container that is configured for enabling a control of an ambient climate within the container and for accommodating one or more boxes for raising worms, the one or more boxes being exposed to the ambient climate within the container,
b) one or more boxes that are configured for accommodating a substrate that is suitable for raising worm(s) and/or worm(s) hatched from cocoon(s),
c) an ambient climate control unit that is configured for adjusting at least one climate parameter to control the ambient climate within the container,
d) a worm detection unit that is configured for automatically determining at least one average property of the, one, two, three or more or all worm(s) present in the substrate, and
e) a ambient climate determination unit that is configured for determining at least one climate parameter in dependence on the determined average property of worms in the substrate for controlling the ambient climate in the container.

8. The system according to claim 7, comprising a collector device that is configured for collecting one, two, three or more or all worm(s) from the substrate.

9. The system according to claim 7 or 8, wherein the worm detection unit comprises an imaging scanner that is configured for generating one or more images, preferably, from several different directions, of the worms present in the substrate.

10. The system according to at least one of claims 7 to 9, wherein the worm detection unit comprises a worm detection artificial neural network that is trained for determining at least one average property of the, one, two, three or more or all worm(s) present in the substrate.

11. The system according to at least one of claims 7 to 10, wherein the ambient climate determination unit comprises an ambient climate artificial neural network that is configured for receiving the least one average property and for providing the at least one climate parameter.

12. The system according to claim 11, comprising a training unit that is configured for training an ambient climate artificial neural network using training data and for providing the trained artificial neural network to the ambient climate determination unit.

13. The system according to at least one of claims 7 to 12, comprising a roller conveyor that is configured for transporting the one or more boxes out of the container and to the worm detection unit for determining the at least one average property of the, one, two, three or more or all worm(s) present in the substrate.

14. A computer program for controlling a temperature and/or humidity and/or pH value of a substrate suitable for raising worm(s) and/or worm(s) hatched from cocoon(s), the computer program including instructions for
- receiving worm detection data indicative of a at least one average property of one or more worm(s),
- determining at least one climate parameter in dependence on the determined average property of worms in the substrate for controlling the ambient climate in the container, and
- providing control instructions for controlling the ambient climate in the container, when run on a computing device.

15. A non-transitory computer readable data medium storing the computer program of claim 14.
